# EUROPEAN PATENT APPLICATION

(11) **EP 1 380 511 A1**
(43) Date of publication of application: **14.01.2004**
(21) Application number: 02425449.2
(22) Date of filing: 08.07.2002
(51) Int. Cl.: B65D 1/02, B65D 1/48, A61J 1/00, A61L 2/26

(54) **Hot-sterilizable plastic bottle**

(71) Applicant: Pagliacci, Gianfilippo, 20145 Milano (IT); Pedulla', Christian Pio, 20124 Milano (IT); Iniziative Generali '96 S.p.A., 34147 Trieste (IT); Tolentino, Luca Pierugo, 20123 Milano (IT)
(72) Inventor: Pagliacci, Gianfilippo, 20145 Milano (IT); Pedulla, Christian Pio, 20124 Milano (IT)
(74) Representative: Faggioni, Marco, Dr. Ing.

(57) **Abstract**

Sterilizable plastic bottle for sterile infusion solutions, of the type sealed by a pierceable rubber plug (4) and a safety metal blocking ring. The bottle (1) is made of plastic material and is provided, at the bottleneck area cooperating with said rubber plug, with a ring (2) of rigid material, substantially free from thermal size modifications, at least within the bottle (1) storage and sterilisation temperature range - varying between -20°C and 120°C - and strictly fitting the external surface of the neck of the bottle (1).

## Description

The present invention relates to a heat sterilizable plastic bottle, and in particular a bottle of this type apt to contain differently composed sterile infusion solutions, to be used for phleboclyses, washings and the like.

In the sanitary industry, glass containers containing sterile solutions to be used for phleboclyses, washings and similar operations are well known and widely employed. Usually, said containers are made of glass and have a 100 cc to 1,000 cc capacity, and a narrow neck plugged with a rubber plug, said plug being blocked by a safety blocking ring of thin metal sheet. The ring prevents the unplugging of the rubber plug, thus ensuring the sterility of the contents, and it has a central removable portion allowing to access, at the moment of usage, the underlying central area of the plug for inserting drugs into the bottle. This plug arrangement allows, in fact, both the insertion of the needle of a plastic infusion tubing, for administering said solution to the patient, and the addition of liquid drugs into the bottle, by means of a common sterile hypodermic syringe pre-filled with the desired drug, whose needle is inserted into the bottle through said rubber plug. The convenience of this container has resulted in a very widespread and global diffusion thereof.

The sterilisation of the bottle and of the solution contained therein is performed - after filling up the bottle, plugging it with the rubber plug and sealing it with the metal blocking ring - by way of a heating process, preferably in an autoclave, at temperatures of over 100°C, so as to ensure the destruction of any pathogenic bacteria or viruses in the liquid or on the bottle or plug walls. To guarantee the preservation of the sterility conditions over the time, the rubber plug has a diameter such as to undergo a predetermined lateral compression when it is inserted into the bottleneck, in order to exert a suitable elastic force against the walls thereof and so prevent any external substance from entering during the manufacturing, transportation, storing and usage of the bottle. Furthermore, the elastomeric material forming the plug is such as to retain its elastic features within the entire temperature range varying from the lowest winter temperatures of a non-heated means of transport or storehouse to sterilisation temperatures, i.e. between -20°C and 120°C approximately.

The manufacturing cost of bottles containing sterile solutions of the above-considered type is strongly influenced by the cost of the glass bottle. In fact, since the bottle contents has a substantially irrelevant cost, it being essentially water, the cost of the final product is mostly determined by the cost of the bottle, of the respective plugging system and, finally, of the bottling and sterilising processes. The plugging system and the bottling and sterilising processes have already been totally optimised and their cost is no longer reducible; the attention of this field's manufacturers has then ever since a long time turned to the possibility of replacing the glass bottles with bottles made of more economical materials, such as plastic.

However, the attempts made so far in this direction have not led to any satisfying or industrially successful results. In fact, although plastic materials with suitable features for use in this particular field have long been available on the market - i.e. plastic materials which do not release toxic or noxious foreign substances and withstand the very wide temperature range indicated above - none of them is apt to guarantee sufficient size stability to maintain the sterility conditions of the bottle contents at the safety degree required by the present laws.

During the sterilisation of the bottle, in fact, a plastic bottle would undergo a significant dilatation, thus changing its overall size. Such a change would have no relevance for the whole body of the bottle, but it would assume a critic importance for the bottleneck. As already specified, in fact, in this area there must be a precise bottleneck-internal-diameter to plug-external-diameter dimensional ratio, in order to ensure that the plug is always suitably compressed, thus exerting a sufficient elastic sealing force against the bottle walls. It is now evident that, during the sterilising process, the bottleneck would undergo a relevant dilatation and subsequent contraction, and this dimensional ratio would thus vary unforeseeably. This change, however, does not only occur during the sterilising same, but it may also persist after the end of the operation, depending on the manner in which the temperature is gradually decreased. Although in most cases, in fact, the contraction of the bottle occurs proportionally to the preceding dilatation-the plug thus resulting suitably compressed in the end-anomalous cooling processes cannot be entirely excluded, wherein the bottleneck may not completely compensate the previous dilatation, or may compensate it asymmetrically, resulting in ovalisation of the bottleneck and subsequent loss of the plug hermetic sealing properties.

If there is even the slightest chance that this kind of phenomena can occur, the complete sterility of the entire bottle production cannot be surely guaranteed. Therefore, despite a growing interest of this field's manufacturers in the replacement of the present glass bottles with plastic bottles, the latter have not had any actual application so far. It must be pointed out that the chance of employing plastic bottles is strongly desirable not only for the lower cost thereof, but also for other advantageous features they could offer, the first of which being lightness and infrangibility.

Considered this high and unanswered demand of the field, the object of the present invention is precisely that of providing a newly conceived plastic bottle which is apt to perfectly preserving sterile solutions for phleboclyses and the like and which may undergo, without incurring in the above listed drawbacks, all the ordinary autoclave sterilisation operations.

This object is obtained, according to the present invention, by means of a bottle having the features indicated in the appended claim 1).

Further features and advantages of the bottle according to the present invention will however be more evident from the following detailed description of some of the preferred embodiments thereof, wherein:

fig. 1 is a schematic isometric view of a first embodiment of the bottle according to the present invention, in a first assembling step;

fig. 2 is a view similar to that of fig. 1, in a second assembling step;

fig. 3 is a view similar to that of fig. 1, wherein the bottle is mounted, before inserting the metal safety blocking ring;

fig. 4 is an enlarged sectional view of the plugging system of the first embodiment of the bottle according to the present invention;

fig. 5 is an enlarged sectional view of the plugging system of a second embodiment of the bottle according to the present invention;

fig. 6 is an enlarged sectional view of the plugging system of a third embodiment of the bottle according to the present invention, and

fig. 7 is an enlarged sectional view of a plugging system of a fourth embodiment of the bottle according to the present invention.

According to the basic feature of the present invention, a bottle 1 - made of a plastic material suitable for the predefined use, i.e., a material which, in particular, does not release any toxic or noxious substances and is apt to withstand the above indicated temperature range, comprised between -20°C and 120°C, without resulting in cracks at low temperatures or structured yielding at high temperatures - is provided at the bottleneck with a seat apt to house a blocking ring 2. The ring 2 is made of a rigid material, substantially free from any significant thermal size modifications within the above indicated temperature range; material suitable for making the ring 2 are: glass, metal, ceramics, plastic materials having a high size stability such as polycarbonates, composite materials, for instance carbon fibres based materials and other materials with similar features.

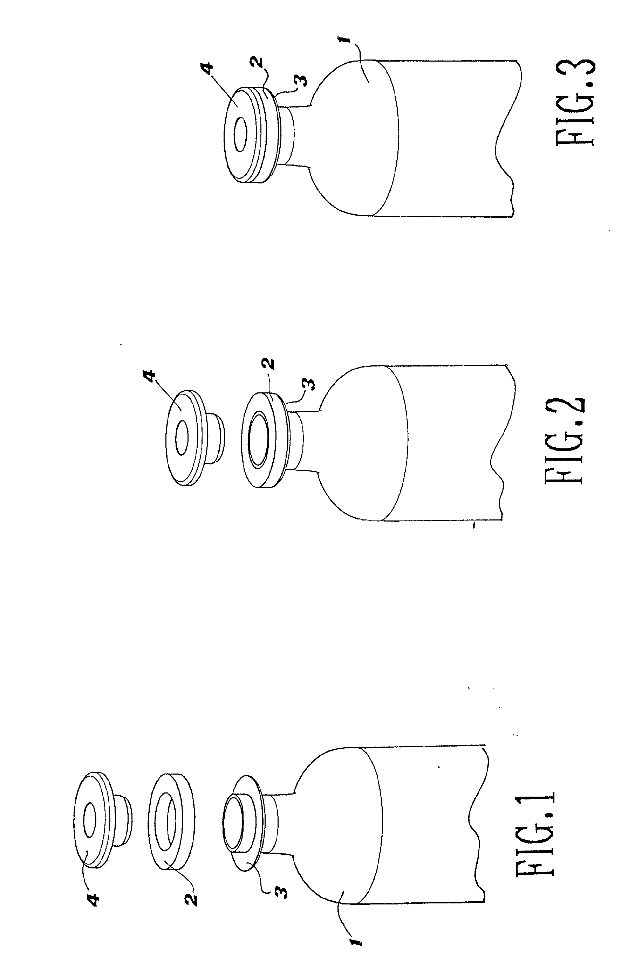

In the first embodiment of the bottle of the present invention, illustrated in figs. 1-4, the seat of the ring 2 is formed by a simple flange 3, projecting from the neck of the bottle 1, onto which the ring 2 is placed, as clearly shown in fig. 2. The internal diameter of the ring 2 is such as to strictly fit the neck of the bottle 1, to which it thus perfectly adheres. After filling up the bottle with the sterile solution, said bottle is plugged in a totally conventional manner with a rubber plug 4 and the sealed bottle looks like the one in fig. 3. Now the bottle is ready to be sealed with the metal safety blocking ring (not shown) and then sent to the sterilisation step.

Although bottle 1 may be subject to even relevant size modifications during the sterilisation process, depending on the type of plastic material from which it is made, the bottleneck, at least in the plug zone, is perfectly stable and free from any modifications in the diameter size, such modifications being prevented by ring 2, which - due to its size steadiness with respect to temperature fluctuations - exerts a powerful and reliable hoping action onto the bottle neck, preventing it from any distortion and thus achieving the object of the invention in a highly simple and sure manner.

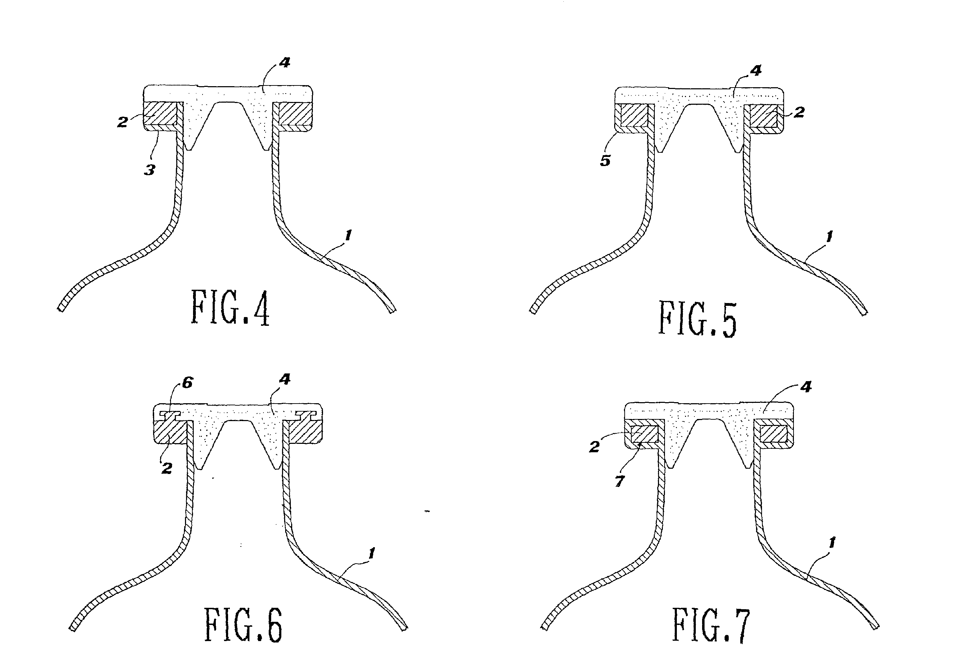

As it is clearly evident, neither the shape and arrangement of the seat of the ring 2, nor its being free, attached to the bottle or, on the contrary, attached to the plug, are of any relevance for reaching the object of the invention. In the following figs. 5, 6 and 7, different possible systems for forming the seat for the ring 2 are shown, which systems should however be understood as merely exemplifying the invention and -not limiting the same. These different embodiments will now be briefly illustrated.

In fig. 5, a second embodiment of the bottle of the present invention is illustrated, wherein the seat of the ring 2 is made of a flange 5 having an L-shaped cross-section, forming therefore, together with the neck of the bottle 1, a seat opened only at the top, into which the ring 2 is forcedly inserted.

In fig. 6, a third embodiment is shown, wherein the seat for the ring 2 is formed within the plug 4. In the lower side of the lateral rim of the plug, in fact, a slot is formed into which a circular rib 6 is inserted, said rib projecting from the upper part of the ring 2 and having a shape apt to perfectly fit the slot provided in the plug 4. In this embodiment, the ring 2 is preferably attached to the plug 4 during the manufacturing of the plug, so as to remain permanently fixed to the plug during the assembling of the bottle.

In fig. 7, a fourth embodiment is shown, wherein the seat for the ring 2 is a completely closed cavity 7, formed in the side wall of the neck of the bottle 1. In other words, the ring 2 is, in this case, included into the neck of the bottle 1 during the moulding of said bottle.

As it is evident from the different embodiments of the bottle described above, irrespective of the different assembly systems, the ring 2 is firmly attached to the external wall of the neck of the body 1, in the zone corresponding to the plug 4, so as to prevent any distortion or ovalisation of said bottleneck during sterilisation at high temperatures.

The present invention has been described with particular reference to some preferred embodiments thereof, but it is evident that the scope of the invention is not limited to such embodiments, but comprises all the possible alternatives within the reach of a person skilled in the art, providing they fall within the scope of the appended claims.

## Claims

1. Sterilizable bottle for sterile infusion solutions, of the type plugged by a pierceable rubber plug (4) and a safety metal blocking ring, **characterised in that** the bottle (4) is made of plastic material and is provided, at the bottleneck zone cooperating with said rubber plug (4), with a ring (2) of a rigid material, substantially free from thermal size modifications, at least within the bottle (1) storage and sterilisation temperature range.

2. Sterilizable bottle as in claim 1), wherein said ring (2) is placed strictly fitting the external surface of the neck of the bottle (1).

3. Sterilizable bottle as in claim 1), wherein said ring (2) is housed in a suitable seat (3, 5) formed in the neck of said bottle (1).

4. Sterilizable bottle as in claim 1), wherein said ring (2) is attached, by way of coupling means (6), to a suitable seat formed in the plug (4) of the bottle (1).

5. Sterilizable bottle as in claim 1), wherein, during the moulding of the bottle (1), said ring (2) is included in a cavity (7) adjacent to the bottleneck.

6. Sterilizable bottle as in any one of the preceding claims, wherein said bottle (1) storage and sterilisation temperature range is comprised between -20°C and 120°C.
